# EUROPEAN PATENT APPLICATION

(11) **EP 3 698 805 A1**
(43) Date of publication of application: **26.08.2020**
(21) Application number: 17928915.2
(22) Date of filing: 20.10.2017
(51) Int. Cl.: A61K 36/758, A23L 33/105, A61K 9/20

(54) **FRACTION OFZANTHOXYLUM PIPERITUM**

(71) Applicant: Mecox Curemed Co., Ltd., Seoul 06744 (KR)
(72) Inventor: KANG, Se Chan, Suwon-si Gyeonggi-do 16710 (KR)
(74) Representative: Viering, Jentschura & Partner mbB Patent- und Rechtsanwälte
(86) International application number: PCT/KR2017/011691
(87) International publication number: WO 2019/078386

(57) **Abstract**

The present invention relates to a composition for pain, containing a fraction obtained by solvent fractionation of a Zanthoxylum piperitum leaf extract. More specifically, a solvent fraction isolated from Zanthoxylum piperitum leaves suppresses all of pain, inflammation and arthritis in a pain-induced animal model, an inflammation-induced model and an arthritis-induced model and, with respect to molecular markers related thereto, exhibits very excellent expression and suppression patterns, thereby being usable in a pharmaceutical composition that is very effective in the treatment of pain- and inflammation-based arthritis.

## Description

### [Technical Field]

The present invention relates to a composition effective for relieving pain containing a fraction of a *Zanthoxylum piperitum* leaf extract.

### [Background Art]

Pain can be divided into primary pain that occurs immediately in response to stimuli and secondary pain that is slowly sensed. Such pain is detected through receptors distributed in the skin or tissues. When the receptors responsible for physical or heat stimulation and chemical stimulation receive and deliver stimulus to the central nervous system, pain is detected. Unlike other sensations, pain becomes more sensitive over time and sensory response to even mild pain increases. Conventional analgesics for treating pain are broadly classified into non-narcotic analgesics and narcotic analgesics. Narcotic analgesics include codeine, morphine, fentanyl and the like and non-narcotic analgesics include acetaminophen, aspirin, ibuprofen, naproxen, Ketoprofen and the like. Non-narcotic analgesics are primarily used as analgesics and anti-inflammatory agents because they inhibit the production of prostaglandin from arachidonic acid and have therapeutic effects on general pain, and pain, fever and inflammation associated with arthritis. Non-narcotic analgesics that are commonly used are generally classified into acetaminophen that generally exhibits analgesic and antipyretic effects and nonsteroidal anti-inflammatory drugs (NSAIDs) that exhibit analgesic, antipyretic and anti-inflammatory effects by inhibiting prostaglandins. Drugs such as acetaminophen are recommended for patients with osteoarthritis not accompanied by inflammation because of the reduced incidence of side effects affecting the stomach, according to guidelines of the American Rheumatology Society. Nonsteroidal anti-inflammatory drugs are recommended to be mainly used for arthritis accompanied by inflammation because of their anti-inflammatory effect, but these non-steroidal drugs should be administered carefully due to the risk of side effects such as gastric ulcers and gastrointestinal bleeding.

Meanwhile, *Zanthoxylum piperitum* is a deciduous shrub of the family Rutaceae, and the fruit bark thereof is generally used as a spice and a medicine. Seeds, young leaves, trees and stems thereof are also used for various purposes. Fruits thereof are used as detoxicants, anthelmintics and analgesics in oriental medicine and are mainly distributed in Korea, Japan and China.

A number of patents on *Zanthoxylum piperitum* have been published. In conventional inventions, research has been conducted mainly on the fruit peel, root and xylem of *Zanthoxylum piperitum,* and *Zanthoxylum piperitum* extracts are known to have effective activity on antibacterial, antiviral, insecticidal, anticancer, anti-inflammatory, arteriosclerosis, hyperlipidemia, diabetes, osteoporosis and skin whitening.

However, there is no document published to date disclosing that the leaf extract of *Zanthoxylum piperitum* is effective in relieving pain.

In particular, the leaf extract of *Zanthoxylum piperitum* contains quercetin-3-O-alpha-L-rhamnoside and camphorol-3-O-alpha-L-rhamnoside serving as both indicators and active compounds. Based on this aspect, it is different from conventional extracts of fruit peel, root and xylem of *Zanthoxylum piperitum.* Furthermore, there is no document disclosing that quercetin-3-O-alpha-L-rhamnoside or camphorol-3-O-alpha-L-rhamnoside is an effective analgesic.

### [Disclosure]

### [Technical Problem]

Thus, as a result of repeated and active research to find a natural substance that is more effective than conventional known anti-inflammatory analgesic-based arthritis treatments, but has low toxicity and few side effects, the present inventors discovered that a leaf extract of *Zanthoxylum piperitum,* a fraction thereof and an active compound isolated therefrom exhibit excellent activities and effects in a pain-induced animal model, an inflammation-induced model, and an arthritis-induced model, thus completing the present invention. In particular, the present inventors discovered that the leaf extract of *Zanthoxylum piperitum* is distinguished from a *Zanthoxylum schinifolium* extract in terms of the great difference in ingredients and has potent analgesic efficacy, and thereby secured the inventive step of the present invention (see Reference Example in Example below).

Therefore, it is one object of the present invention to provide a pharmaceutical composition for treating and preventing pain containing, as an active ingredient, a leaf extract of *Zanthoxylum piperitum,* a fraction thereof or an active compound isolated therefrom.

It is another object of the present invention to provide a health food composition for relieving pain containing, as an active ingredient, a leaf extract of *Zanthoxylum piperitum,* a fraction thereof, or an active compound isolated therefrom.

It is another object of the present invention to provide a method of isolating an active compound from leaves of *Zanthoxylum piperitum.*

### [Technical Solution]

In accordance with an aspect of the present invention, the above and other objects can be accomplished by the provision of a pharmaceutical composition for treating and preventing pain containing, as an active ingredient, quercetin-3-O-alpha-L-rhamnoside represented by the following Formula 1, camphorol-3-O-alpha-L-rhamnoside represented by the following Formula 2, or a mixture thereof, wherein the pharmaceutical composition comprises a *Zanthoxylum piperitum* extract obtained by extracting *Zanthoxylum piperitum* leaves with at least one solvent selected from C₁₋₄ alcohol and a C₁₋₄ alcoholic aqueous solution.

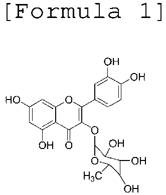

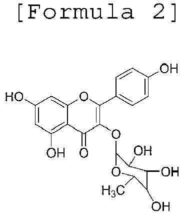

In accordance with another aspect of the present invention, there is provided a pharmaceutical composition for treating and preventing pain containing an ethyl acetate fraction obtained by extracting the *Zanthoxylum piperitum* extract with ethyl acetate.

In accordance with another aspect of the present invention, there is provided a pharmaceutical composition for treating and preventing pain containing at least one compound selected from the group consisting of (a) quercetin-3-O-alpha-L-rhamnoside, (b) camperol-3-O-alpha-L-rhamnoside, (c) 4'-hydroxy-acetophenone, (D) 2,4-di-tert-butylphenol and (E) 1,2-benzenedicarboxylic acid.

In accordance with another aspect of the present invention, there is provided a health food composition for relieving pain containing, as an active ingredient, quercetin-3-O-alpha-L-rhamnoside represented by Formula 1, camphorol-3-O-alpha-L-rhamnoside represented by Formula 2, or a mixture thereof, wherein the health food composition comprises a *Zanthoxylum piperitum* extract obtained by extracting *Zanthoxylum piperitum* leaves with at least one solvent selected from C₁₋₄ alcohol and a C₁₋₄ alcoholic aqueous solution.

In accordance with another aspect of the present invention, there is provided a health food composition for relieving pain containing an ethyl acetate fraction obtained by extracting the *Zanthoxylum piperitum* extract with ethyl acetate.

In accordance with another aspect of the present invention, there is provided a health food composition for relieving pain containing at least one compound selected from the group consisting of (a) quercetin-3-O-alpha-L-rhamnoside, (b) camperol-3-O-alpha-L-rhamnoside, (c) 4'-hydroxy-acetophenone, (D) 2,4-di-tert-butylphenol and (E) 1,2-benzenedicarboxylic acid.

In accordance with another aspect of the present invention, there is provided a method of isolating an active compound from *Zanthoxylum piperitum,* including:
a) extracting leaves of *Zanthoxylum piperitum* with at least one extraction solvent selected from C₁₋₄ alcohol and a C₁₋₄ alcohol aqueous solution to obtain an alcohol extract;
b) suspending the alcohol extract with water and then fractionating the resulting suspension with normal hexane, dichloromethane, ethyl acetate and normal butanol in sequence to obtain an ethyl acetate fraction; and
c) subjecting the ethyl acetate fraction to silica gel column chromatography and medium-pressure liquid chromatography (MPLC) to form quercetin-3-O-alpha-L-rhamnoside represented by Formula 1 or camperol-3-O-alpha-L-rhamnoside represented by Formula 2.

### [Advantageous effects]

The leaf extract of *Zanthoxylum piperitum,* the fraction thereof and the active compounds isolated therefrom provided by the present invention, which are quercetin-3-O-alpha-L-rhamnoside and camphorol-3-O-alpha-L-rhamnoside, exhibited excellent therapeutic efficacies upon testing using pain-induced animal models, inflammation-induced models, and arthritis-induced models.

Accordingly, the leaf extract of *Zanthoxylum piperitum,* the fraction thereof and the active compound isolated therefrom can be used as an effective ingredient for pharmaceuticals or health foods for treating, preventing or relieving pain-associated diseases.

### [Brief Description of the Drawings]

FIG. 1 is a schematic diagram showing a process for obtaining a solvent fraction and an active compound from a *Zanthoxylum piperitum* leaf extract.

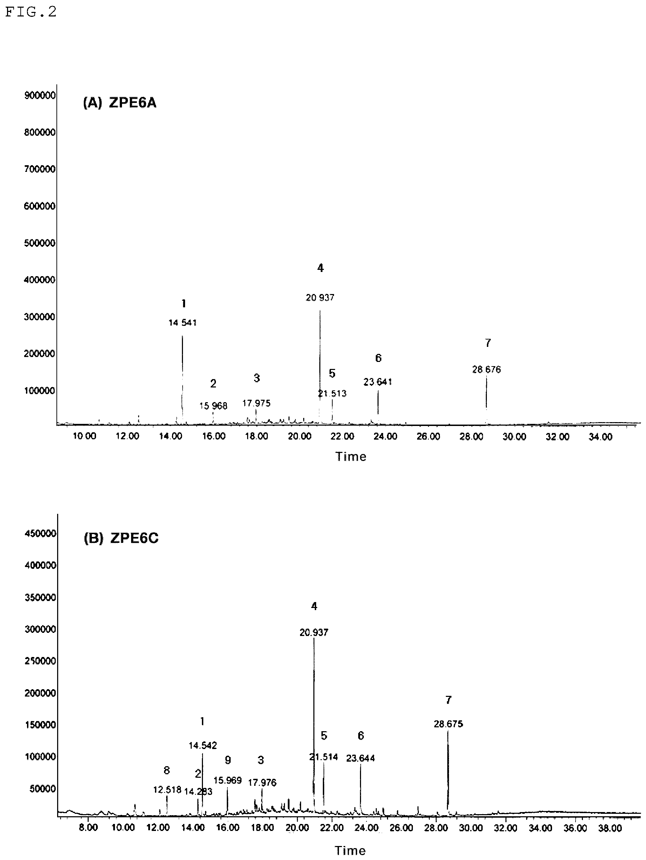

FIG. 2 is a GC-MS spectrum of (A) ZPE6A and (B) ZPE6C subfractions.
FIG. 3 is a graph showing a pain-relieving effect of 4'-hydroxyacetophenone, 2,4-di-tert-butylphenol, and 1,2-benzenedicarboxylic acid isolated from a ZPE6A or ZPE6C fraction.
FIG. 4 is a graph showing a pain-inhibiting effect of the *Zanthoxylum piperitum* extract identified using (a) a tail-flick test, (b) a hot-plate test, and (c) a formalin test.
FIG. 5 shows a BDNF expression pattern in BV-2 microglia of the *Zanthoxylum piperitum* leaf extract measured using enzyme immunoassay.
FIG. 6 is a graph showing the effect of the *Zanthoxylum piperitum* leaf extract on inhibition of the expression of inflammatory markers iNOS, COX-2, TNF-α, IL-1β and IL-6 identified using (A) reverse transcription-PCR analysis and (B) immune-blotting.
FIG. 7 shows the result of identification of the anti-inflammatory effect of the *Zanthoxylum piperitum* leaf extract in a subchronic ear edema model.
FIG. 8 is a 3D image showing the therapeutic effect of rheumatoid arthritis by the *Zanthoxylum piperitum* leaf extract identified in a collagen-induced rheumatoid arthritis model (CIA).
FIG. 9 is an H&E staining image showing the therapeutic effect of osteoarthritis of the *Zanthoxylum piperitum* leaf extract identified in a collagenase-induced osteoarthritis model.
FIG. 10 shows changes in biochemical indicators of (A) MMP-2, (B) MMP-3, (C) TIMP-1 and (D) TIMP-2 with respect to the *Zanthoxylum piperitum* leaf extract, quercetin-3-O-alpha-L-rhamnoside and camphorol-3-O-alpha-L-rhamnoside each identified in the osteoarthritis model.
FIG. 11 shows an expression level of K+ gate regulatory protein (KCNJ6) in BV-2 microglia after treatment with the *Zanthoxylum piperitum* leaf extract, quercetin-3-O-alpha-L-rhamnoside or camphorol-3-O-alpha-L-rhamnoside.
FIG. 12 is a graph showing a potassium (K⁺) current density measured in BV-2 microglia.
FIG. 13 is a graph showing an expression level of IFN-γ measured in NK cells after treatment with the *Zanthoxylum piperitum* leaf extract, quercetin-3-O-alpha-L-rhamnoside and camphorol-3-O-alpha-L-rhamnoside in a collagen-induced animal model (CIA).
FIG. 14 is a graph showing expression levels of inflammation-promoting cytokines (A: IL-6, B: IL-17, C: IL-21 and D: IL-22) in T cells and TH17 cells after treatment of the collagen-derived animal model (CIA) with the *Zanthoxylum piperitum* leaf extract quercetin-3-O-alpha-L-rhamside and camphorol-3-O-alpha-L-rhamnoside.

### [Best Mode]

The present invention will be described in more detail as follows.

The present invention is directed to a composition for relieving, preventing and treating pain containing, as an active ingredient, a leaf extract of *Zanthoxylum piperitum,* a solvent fraction obtained by extracting the extract with an organic solvent, and quercetin-3-O-alpha-L-rhamnoside or camphorol-3-O-alpha-L-rhamnoside isolated therefrom.

Also, the present invention is directed to a method including a series of steps for isolating an extract, a fraction and an active compound from *Zanthoxylum piperitum,* and the method specifically includes the following steps:
a) extracting leaves of *Zanthoxylum piperitum* with at least one extraction solvent selected from C₁₋₄ alcohol and a C₁₋₄ alcohol aqueous solution to obtain an alcohol extract;
b) suspending the alcohol extract with water and then fractionating the resulting suspension with normal hexane, dichloromethane, ethyl acetate and normal butanol in sequence to obtain an ethyl acetate fraction; and
c) subjecting the ethyl acetate fraction to silica gel column chromatography and medium-pressure liquid chromatography (MPLC) to form quercetin-3-O-alpha-L-rhamnoside represented by Formula 1 or camperol-3-O-alpha-L-rhamnoside represented by Formula 2.

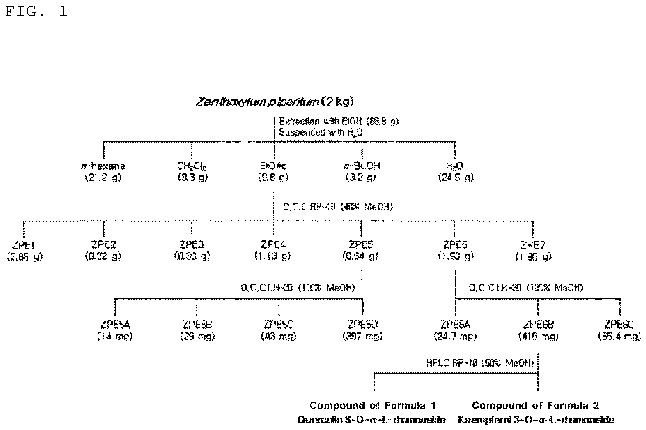

The method of isolating the extract, solvent fraction or active compound from leaves of *Zanthoxylum piperitum* will be described in more detail with reference to the schematic diagram of FIG. 1.

Step a) is to obtain a *Zanthoxylum piperitum* leaf extract.

The entire *Zanthoxylum piperitum* plant, including leaves, stems, branches and roots, can be used. According to the results of experimentation by the present inventors, there was a great difference in the pain inhibition activity between respective parts of *Zanthoxylum piperitum.* More specifically, the *Zanthoxylum piperitum* leaf extract had about 700% higher pain inhibition percentage than a *Zanthoxylum piperitum* root extract, and about 26% higher pain inhibition percentage than a *Zanthoxylum piperitum* fruit extract [See Example 1]. In the present invention, a leaf part is selected and used from among various parts of *Zanthoxylum piperitum.*

The leaves of *Zanthoxylum piperitum* are washed with water, dried, and then further dried in the shade. The leaves of *Zanthoxylum piperitum* are immersed and extracted in at least one extraction solvent selected from C₁₋₄ alcohol and a C₁₋₄ alcohol aqueous solution. The extraction solvent may be selected from methanol, ethanol, isopropanol, propanol, butanol, and an aqueous solution thereof, preferably ethanol or an ethanol aqueous solution, and more preferably an aqueous ethanol solution of 90 to 95% concentration. The extraction solvent is preferably used in an amount of 1:1 to 1:30 by weight, more preferably 1:10 to 1:20 by weight with respect to the dry weight of the leaves of *Zanthoxylum piperitum,* but the present invention is not limited as to the amount of the extraction solvent. The temperature at the time of extraction may range from room temperature to the reflux temperature of the extraction solvent, and specifically may be a temperature of 20 to 80°C. The extraction time is preferably 1 to 10 days, but is not limited thereto. In addition, the extraction may be carried out one or more times. However, since the yield of the active ingredient is significantly reduced as extraction continues, it may not be economical to repeat extraction more than five times. Thus, it is preferable to perform the extraction two to five times.

The extraction method may be a method commonly known in the art, for example, a method using an extraction device such as supercritical extraction, subcritical extraction, high-temperature extraction, high-pressure extraction, or ultrasonic extraction, or a method using a an adsorption resin including XAD and HP-20.

Additionally, the extract is obtained by concentration under reduced pressure using a vacuum rotary evaporator or the like. In addition, the obtained extract may be subjected to reduced-pressure drying, vacuum drying, boiling drying, spray drying, roomtemperature drying or freeze drying, if necessary.

Step b) is to extract *Zanthoxylum piperitum* with an organic solvent to obtain an ethyl acetate fraction.

Specifically, the ethanolic extract of *Zanthoxylum piperitum* obtained in step a) is suspended in water and then series-fractionated with normal hexane, dichloromethane, ethyl acetate and normal butanol in sequence to obtain fractions. Among the fractions, an ethyl acetate fraction having the highest activity is obtained. The fractionation may be repeated once to five times, preferably three times, and may include concentration under reduced pressure and drying after the fractionation, but the present invention is not limited thereto.

Step c) is to subject the ethyl acetate fraction to silica gel column chromatography and medium-pressure liquid chromatography (MPLC) to separate and obtain an active compound.

Specifically, silica gel column chromatography is performed by charging the ethyl acetate fraction obtained in step b) into a glass column (5 x 50 cm) filled with silica beads. At this time, a mixed solution of ethyl acetate and methanol was used as the eluent, and the mixture was eluted while changing the volume ratio of ethyl acetate/methanol from 90/10 to 60/40. As a result of the silica gel column chromatography, seven fractions were obtained, and the resulting fractions were designated "ZPE1", "ZPE2", "ZPE3", "ZPE4", "ZPE5", "ZPE6", and "ZPE7". Among the seven fractions obtained through the silica gel column chromatography, ZPE6 showed the best activity. The ZPE6 fraction was obtained and subjected to medium-pressure liquid chromatography (MPLC).

The selected ZPE6 fraction was charged in a Sephadex LH-20 column and subjected to medium-pressure liquid chromatography (MPLC). At this time, a methanol solution was used alone as an eluent. As a result of the medium-pressure liquid chromatography (MPLC), three sub-fractions were obtained. The resulting sub-fractions were designated "ZPE6A", "ZPE6B", and "ZPE6C". Among the three sub-fractions obtained by medium-pressure liquid chromatography (MPLC), ZPE6B showed the best activity. The ZPE6B sub-fraction was selected and the active compound was isolated therefrom.

In order to separate the active compound from the ZPE6B sub-fraction, high-performance liquid chromatography (HPLC) was performed using a reverse-phase column filled with octadecylated silica. At this time, a mixed solution of methanol and water was used as an eluent. Preferably, a 50% methanol aqueous solution containing equivalent amounts of methanol and water is used.

Nuclear magnetic resonance spectroscopy identified that the active compound separated through HPLC is a quercetin-3-O-alpha-L-rhamnoside (Quercitrin) represented by the following Formula 1 and camphorol-3-O-alpha-L-rhamnoside (Afzelin) represented by the following Formula 2.

The chemical structures of the active ingredient 1 and the active ingredient 2 separated above were identified through nuclear magnetic resonance spectroscopy.

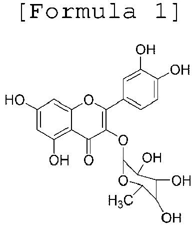

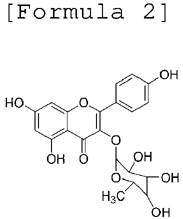

The *Zanthoxylum piperitum* leaf extract obtained through the separation method, the solvent fractions obtained by extraction with the organic solvent and the quercetin-3-O-alpha-L-rhamnoside (Formula 1) and camphorol-3-O-alpha-L-rhamnoside (Formula 2) separated therefrom were found to exhibit significant results in the pain-induced animal model, the inflammation-induced animal model and the arthritis-induced animal model. Therefore, the *Zanthoxylum piperitum* leaf extract, the fraction thereof and/or the compound represented by Formula 1 or 2 as an active compound separated therefrom is contained as an effective (active) ingredient in a pharmaceutical composition or health food composition for alleviating, preventing and treating pain and inflammatory diseases.

The pharmaceutical composition according to the present invention includes the *Zanthoxylum piperitum* leaf extract, the fraction thereof and/or the active compound separated therefrom as an effective (active) ingredient, and the effective (active) ingredient may be present in an amount of 0.1 to 50% by weight based on the total weight of the composition, but the present invention is not limited thereto.

The pharmaceutical composition of the present invention may be used as an oral formulation such as a powder, a granule, a tablet, a capsule, a suspension, an emulsion, a syrup or an aerosol, or a formulation in the form of an external preparation, a suppository or a sterile injection solution according to a conventional method. Therefore, the pharmaceutical composition of the present invention may further include a suitable carrier, excipient and diluent commonly used in the preparation of drugs.

The carrier, excipient and diluent which may be included in the pharmaceutical composition of the present invention include at least one selected from lactose, dextrose, sucrose, sorbitol, mannitol, xylitol, erythritol, maltitol, starch, acacia rubber, alginate, gelatin, calcium phosphate, calcium silicate, cellulose, methyl cellulose, hydroxymethyl cellulose, microcrystalline cellulose, silicified microcrystalline cellulose, povidone, crospovidone, croscarmellose sodium, polyvinylpyrrolidone, water, methylhydroxybenzoate, propylhydroxybenzoate, Neusilin, colloidal silicon dioxide, lactose, talc, magnesium stearate, colloidal stearyl magnesium and mineral oil.

When formulated, diluents or excipients such as fillers, extenders, binders, wetting agents, disintegrating agents and surfactants are usually used. Solid preparations for oral administration include tablets, pills, powders, granules, troches, rosin, capsules and the like, and such solid preparations are prepared by mixing the composition of the present invention with at least one excipient, for example, lactose, saccharose, sorbitol, mannitol, starch, amylopectin, cellulose, calcium carbonate, gelatin and the like. In addition to simple excipients, lubricants such as magnesium stearate and talc are also used. Liquid preparations for oral administration include suspensions, liquids for internal use, emulsions, elixirs, and syrups. In addition to commonly used simple diluents such as water and liquid paraffin, various excipients such as wetting agents, sweeteners, fragrances and preservatives may be included. Formulations for parenteral administration include sterile aqueous solutions, non-aqueous solvents, suspensions, emulsions, lyophilized preparations, and suppositories. As the non-aqueous solvent and suspension agent, propylene glycol, polyethylene glycol, vegetable oil such as olive oil, injectable ester such as ethyl oleate or the like can be used. As a base of suppositories, Witepsol, macrogol, tween 61, cacao butter, laurin butter, glycerol gelatin or the like may be used. Parenteral administration may generally be subcutaneous, intravenous, intramuscular or intrathoracic injection.

The pharmaceutical composition of the present invention may be administered orally or parenterally, any parenteral administration method may be used, and systemic or topical administration is possible, but systemic administration is further preferred, and intravenous administration is most preferred.

The preferred dosage of the pharmaceutical composition of the present invention varies depending on the condition and weight of the patient, the severity of the disease, the form of the drug, and the route and duration of administration and may be appropriately selected by those skilled in the art. However, for the preferred effect, the pharmaceutical composition of the present invention is preferably administered at 0.0001 to 1,000 mg/kg, preferably at 10 to 300 mg/kg (weight). Administration may be carried out once or several times a day. The dosage does not limit the scope of the invention in any aspect.

In addition, the health food composition according to the present invention includes the *Zanthoxylum piperitum* leaf extract, the fraction thereof and/or the active compound separated therefrom as an effective (active) ingredient, and may be added to health supplements such as foods and beverages.

There is no particular limitation as to the kind of food. Examples of foods to which the effective ingredient can be added include drinks, meats, sausages, bread, biscuits, rice cakes, chocolate, candy, snacks, confectionaries, pizza, ramen, other noodles, gum, dairy products including ice cream, various soups, beverages, alcoholic beverages and vitamin complexes, dairy and processed dairy products, and includes all health functional foods in the conventional sense. The effective ingredient may be added to a food as it is or used in conjunction with other food or food ingredients, and may be appropriately used according to a conventional method. The content of the effective ingredient can be suitably determined according to the purpose of use (prevention or alleviation). In general, the active ingredient may be present in an amount of 0.1 to 90% by weight of the health food composition. However, in the case of long-term administration for health and hygiene or health control, the amount may be below the range defined above, and the active ingredient may be used in an amount exceeding the above range because there is no problem in terms of safety.

A beverage can be prepared using the health food composition of the present invention. There is no particular limitation as to the selection of ingredients other than the effective ingredient as the ingredient included in the beverage, and various flavoring agents, natural carbohydrates or the like may be added as additional ingredients, like general drinks. Examples of natural carbohydrates include conventional sugars, for example monosaccharides such as glucose and fructose, disaccharides such as maltose and sucrose, polysaccharides such as dextrin and cyclodextrin, and sugar alcohols such as xylitol, sorbitol and erythritol. As flavoring agents other than those mentioned above, natural flavoring agents (thaumatin, stevia extract (e.g., rebaudioside A or glycyrrhizin) and synthetic flavoring agents (saccharin or aspartame) can be advantageously used. The natural carbohydrate is generally used in an amount of about 1 to 20 g, and preferably about 5 to 12 g with respect to 100 mL of the composition of the present invention.

In addition, the health food composition of the present invention, in addition to the active ingredient, may include, as additives, various nutrients, vitamins, minerals (electrolytes), flavoring agents such as synthetic flavoring agents and natural flavoring agents, coloring and neutralizing agents (such as cheese and chocolate), pectic acid and salts thereof, alginic acid and salts thereof, organic acids, protective colloidal thickeners, pH adjusters, stabilizers, preservatives, glycerin, alcohols, carbonation agents used in carbonated beverages and the like. In addition, the health food composition may contain flesh for the production of natural fruit juices, fruit juice drinks and vegetable drinks. These ingredients may be used independently or in combination. The content of such an additive is not critical, but is generally selected within the range of 0.1 to 20% by weight of the health food composition of the present invention.

### [Mode for Invention]

Hereinafter, the present invention will be described in more detail with reference to the following examples. However, the following examples should not be construed as limiting the scope of the present invention.

### [Example]

### [Measurement of pain inhibition effect and anti-inflammatory effect]

The pain inhibition effect and anti-inflammatory effect of the *Zanthoxylum piperitum* leaf extract, fraction or active compound obtained in the present invention were measured by the following method.

### 1. Identification of pain inhibition effect

### (1) Analgesic effect by acetic-acid-induced pain model

The acetic acid-induced writhing syndrome test is an animal model for determining analgesic effects. It is mainly used to test the effect of analgesics on the peripheral nervous system. The experimental animals used herein were 5-week-old ICR mice purchased from Central Laboratory Animals (Seoul) and acclimated in a breeding room maintained at a humidity of 50% and a temperature of 24 to 26°C for one week. Feed and water were supplied for free intake. When 1.5% acetic acid was injected into the abdominal cavity of mice as an irritant, it caused pain in the abdominal cavity, thus causing mice to exhibit a writhing syndrome reaction, for example, twisting the body or stretching the hind legs. The test substance was orally administered 60 minutes before the induction of pain to determine the degree of analgesic effect.

### (2) Analgesic effect by tail-flick test

The tail-flick test is mainly used to test the effects of analgesics on the central nervous system using acute pain models. The test substance was orally administered 30 minutes and 60 minutes before the induction of pain, and then the tail of the mouse was placed on a hot plate and the tail avoidance response was measured.

### (3) Analgesic effect by hot-plate test

The hot-plate test is used to search for analgesics acting on the central nervous system. Since the sole of the mouse is very sensitive to heat, the rat jumps, or shrinks or licks the sole when detecting unbearable heat. After oral administration of the test substance 60 minutes before the induction of pain, the time (sec) from when the rat contacted the hot plate to when the mouse shook or licked the hind legs was measured.

### (4) Analgesic effect by formalin test

The formalin test is primarily used to measure the analgesic effects of drugs in chronic pain models, in which the pain stimulus and response are not temporary but persistent. The pain response induced by formalin includes phase 1 and phase 2, and these two phases can be distinguished pharmacologically. The test substance and acetaminophen or aspirin, as a positive control group, were orally administered 60 minutes before the induction of pain, 10% formalin was injected subcutaneously into the hind legs of rats, and the number of actions in response to pain was measured every 3 minutes. The action in response to pain was measured separately in phase 1 (0-12 minutes) and phase 2 (24-39 minutes).

### 2. Identification of anti-inflammatory efficacy

### (1) Enzyme-linked immunosorbent assay

A brain-derived neurotrophic factor (BDNF) is a neurotrophic factor that is present mainly in the brain, that binds to receptors such as tropomyosin-related kinase B (TrkB) and p75, and that causes pain through interaction with these receptors. In order to measure the BDNF expression behavior caused by the test substance, the amount of BDNF expression in BV-2 microglia was examined using an ELISA kit (Uscn, china).

### (2) Reverse Transcription-PCR Assay

RNA was lysed using a TRIzol Reagent kit (Gibco BRL) according to the manufacturer's instructions. 1 µg of RNA was reverse transcribed with 10,000 U of a reverse transcriptase and 0.5 g/L of oli-(dT)15 primer. 1 µL of synthesized cDNA was amplified by PCR using iNOS, COX-2, TNF-α, IL-1β and IL-6 primers. PCR products were separated on 1% agarose gels and visualized under UV radiation.

### (2) Immunoblot analysis

Cell lysates were immersed in 40 µL of buffer solution and stored for 30 minutes at 4°C, and then 30 µg of protein was isolated by electrophoresis on a 10% SDS-polyacrylamide gel. The protein was then transferred to nitrocellulose cell membranes and blocked with skim milk powder for 1 hour. The protein was washed three times with TBST buffer for 10 minutes and treated with a primary antibody. Cell membranes were stored with a secondary antibody bound to horseradish peroxidase (HRP). Immunoreactive bands were detected by ECL enhanced chemiluminescence reagents according to the manufacturer's instructions.

### (4) Anti-inflammatory effect in subchronic ear edema model

An anti-inflammatory effect was measured in a mouse ear edema model in which ear edema was induced by 12-O-tetradecanoyl-13-phorbol acetate (TPA). A test substance was orally administered to five-week-old ICR mice that had undergone a one-week acclimation process. After 1 hour, TPA was applied in an amount of 1 µg/ear to the right ear. This process was repeated for 3 days, the thickness of the mouse ear was measured on the fourth day, and the weight per area of ear tissue was measured.

### [Production example]

### Preparation Example 1. Isolation of Zanthoxylum piperitum DC leaf extract, fraction and active compound

The process of separating an extract, a fraction and an active compound from *Zanthoxylum piperitum* DC leaves will be described with reference to the schematic process diagram shown in FIG. 1.

*Zanthoxylum piperitum* DC was a domestic product obtained from the southern area of South Korea. The leaves of *Zanthoxylum piperitum* DC were washed, dried, and then further dried in the shade. The leaves of *Zanthoxylum piperitum* DC were immersed in a 90% ethanol aqueous solution and extracted 2 to 5 times at room temperature. The extract was distilled under reduced pressure to obtain an ethanol extract of *Zanthoxylum piperitum* DC leaves.

68.8 g of the ethanol extract of *Zanthoxylum piperitum* DC leaves was suspended in 2L of distilled water, sequentially fractionated with equal amounts of normal hexane, dichloromethane, ethyl acetate, normal butanol and water, and then concentrated under reduced pressure to obtain each solvent fraction. For each solvent fractions obtained, the anti-inflammatory and pain inhibition effects were determined, and among them, the ethyl acetate fraction showing the best activity was selected.

The ethyl acetate fraction was charged in a glass column (5 x 50 cm) filled with silica beads and subjected to silica gel column chromatography. At this time, a mixed solution of ethyl acetate and methanol was used as the eluent, and the mixture was eluted while changing the volume ratio of ethyl acetate/methanol from 90/10 to 60/40. Silica gel column chromatography was performed to obtain seven fractions (ZPE1, ZPE2, ZPE3, ZPE4, ZPE5, ZPE6, ZPE7). The times at which the ingredient having an identical Rf appears according to the fractionation time (eluted every two minutes) and the yields of the obtained seven fractions (MPLC, eluant, ethyl acetate/methanol volume ratio, 90:10 to 60:40) are summarized in Table 1 below.

**[Table 1]**

| Sub-fraction of ethyl acetate fraction | | |
|---|---|---|
| Item | Elution fraction time | Yield (g) |
| fraction ZPE1 | 0 ∼ 80 min | 2.86 |
| fraction ZPE2 | 81 ∼ 100 min | 0.32 |
| fraction ZPE3 | 101 ∼ 120 min | 0.30 |
| fraction ZPE4 | 121 ∼ 160 min | 1.13 |
| fraction ZPE5 | 161 ∼ 178 min | 0.54 |
| fraction ZPE6 | 179 ∼ 210 min | 1.90 |
| fraction ZPE7 | 211 ∼ 250min | 1.90 |

The anti-inflammatory and pain inhibition effects of each of the seven fractions were determined, and among them, the ZPE6 fraction showing the best activity was selected. The ZPE6 fraction was concentrated under reduced pressure, dried completely, and charged in a Sephadex LH-20 column to perform medium-pressure liquid chromatography (MPLC). At this time, a solution of methanol alone was used alone as the eluate, and the eluate was collected at 10-minute intervals and identified by TLC. Only the portions at which points having an identical Rf value were formed were combined to obtain a sub-fraction. Medium-pressure liquid chromatography (MPLC) was performed to obtain three sub-fractions (ZPE6A, ZPE6B and ZPE6C). The following Table 2 shows the fractionation time and yield of the three obtained fractions.

**[Table 2]**

| Sub-fraction of fraction ZPE6 | | |
|---|---|---|
| Item | Elution fraction time | Yield (mg) |
| fraction ZPE6A | 55 ∼ 145 min | 24.7 |
| (fraction ZPE6B | 146 ∼ 280 min | 416 |
| fraction ZPE6C | 281 ∼ 360 min | 85.4 |

The anti-inflammatory and pain inhibition effects of each of the three sub-fractions were determined, and among them, the ZPE6B sub-fraction having the best activity was selected.

The sub-fraction ZPE6B was subjected to HPLC (eluent: methanol/water = 50/50) using a reverse-phase column filled with octadecylated silica, and active ingredient 1 and active ingredient 2 were separated.

The chemical structures of active ingredient 1 and active ingredient 2 separated above were identified through chemical magnetic resonance spectroscopic analysis.

### Active Ingredient 1:

¹H-NMR (300 MHz, DMSO- *d*₆) δ 0.9 (d, J = 6 Hz, CH ₃), 5.2 (d, J = 1.5 Hz, H-1"), 6.15 (d, J = 2.2 Hz, H-6), 6.36 (d, *J* = 2.2 Hz, H-8), 6.95 (d, *J* = 8.2 Hz, H-5'), 7.25 (dd, J = 2.2 Hz and *J* = 8.2 Hz, H-6'), 7.35 (d, J = 2.2 Hz, H-2'); ¹³C-NMR (75 MHz, DMSO- *d*₆) δ 17.5 (C-6"), 70.0 (C-5"), 70.2 (C-3"), 70.5 (C-2"), 71.1 (C-4"), 93.6 (C-8), 98.6 (C-6), 101.8 (C-1"), 104.0 (C-10), 115.4 (C-2'), 115.6 (C-5'), 120.7 (C-6'), 121.1 (C-1'), 134.2 (C-3), 145.1 (C-3'), 148.3 (C-4'), 156.4 (C-2), 157.2 (C-9), 161.3 (C-5), 164.2 (C-7), 177.7 (C-4)

Based on the result of spectroscopic analysis, the active ingredient 1 was identified as quercetin-3-O-alpha-L-rhamnoside, represented by the following Formula 1. The results of spectroscopic analysis on active ingredient 1 corresponded well to the literature values for quercetin-3-O-alpha-L-rhamnoside (Quercitrin).

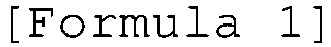

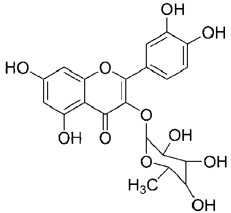

### Active ingredient 2:

¹H-NMR (300 MHz, DMSO-*d*₆) δ 0.9 (d, J = 6 Hz, CH₃), 5.29 (1H, d, J = 1.5 Hz, H-1"), 6.21 (1H, d, J = 2.5 Hz, H-6), 6.42 (1H, d, J = 2.5 Hz, H-1"), 6.21 (1H, d, J = 2.5 Hz, H-6), 6.42 (1H, d, J = 2.5 Hz, H-8), 6.91 (2H, d, J = 8.4 Hz, H-3' and H-5'), 7.74 (2H, d, J = 8.4 Hz, H-2' and H-6'); ¹³C-NMR (75 MHz, DMSO- *d*₆) δ 17.4 (C-6"), 70.0 (C-5"), 70.2 (C-2"), 70.6 (C-3"), 71.0 (C-4"), 93.7 (C-8), 98.7 (C-6), 101.7 (C-1"), 104.0 (C-10), 115.3 (C-3', C-5'), 120.5 (C-1), 130.6 (C-2', C-6'), 134.2 (C-3), 156.5 (C-9), 157.2 (C-2), 159.9 (C-4'), 161.2 (C-5), 164.3 (C-4), 177.7 (C-7)

Based on the result of spectroscopic analysis, the active ingredient 2 was identified as camphorol-3-O-alpha-L-rhamnoside, represented by the following Formula 2. The results of spectroscopic analysis of active ingredient 1 corresponded well to the literature values for camphorol-3-O-alpha-L-rhamnoside (Afzelin).

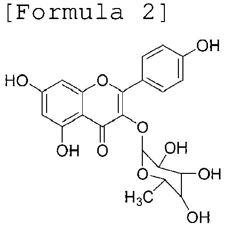

### Example 1. Comparison of pain inhibition effect between parts of Zanthoxylum piperitum

In order to compare the pain-suppressing effect of the extracts between respective parts of *Zanthoxylum piperitum,* namely the leaves, young branches, fruits, stems and roots thereof, a 90% ethanol extract was obtained by the extraction method according to Preparation Example 1. For each extract, the pain inhibition effect was measured using an acetic acid-induced pain model, and the results are shown in Table 3 below.

**[Table 3]**

| Pain inhibition percentage of ethanol extract depending on part of *Zanthoxylum piperitum* | |
|---|---|
| *Zanthoxylum piperitum* part | Pain inhibition percentage (%)(100 mg/kg, po) |
| leaves | 91.6 |
| young branches | 84.7 |
| fruits | 72.8 |
| stems | 65.0 |
| roots | 11.8 |

The results of Table 3 showed that there was a marked difference in the pain inhibition effect between parts of *Zanthoxylum piperitum,* and that the efficacy of the leaf extract was the most excellent. It can be seen that the leaf extract has about a 25.8% higher pain inhibition percentage than the fruit extract.

### Example 2. Comparison of pain inhibition effect between concentrations of aqueous ethanol solution

The pain inhibition effect of the *Zanthoxylum piperitum* leaf extract was compared according to the concentration of the extract solution. That is, ethanol extracts of *Zanthoxylum piperitum* leaves were obtained using an ethanol aqueous solution with a concentration of 0 to 100% by the extraction method according to Preparation Example 1. In addition, the pain inhibition effect of each extract was measured using an acetic acid-induced pain model, and the results are shown in Table 4 below.

**[Table 4]**

| Pain inhibition percentage of *Zanthoxylum piperitum* leaf extract depending on concentration of ethanol aqueous solution | |
|---|---|
| Concentration of ethanol aqueous solution (%) | Pain inhibition percentage (%) (100 mg/kg, po) |
| 0 (water) | 30.8 |
| 10 | 31.8 |
| 20 | 36.4 |
| 30 | 38.3 |
| 40 | 40.2 |
| 50 | 41.1 |
| 60 | 43.3 |
| 70 | 55.1 |
| 75 | 55.1 |
| 80 | 57.9 |
| 85 | 71.0 |
| 90 | 91.6 |
| 95 | 90.1 |
| 100 | 85.4 |

The results of Table 4 showed that, as the concentration of the ethanol aqueous solution used for extraction increased, the pain-inhibition percentage of the *Zanthoxylum piperitum* leaf extract increased. In terms of the pain inhibition effect, the concentration of the ethanol aqueous solution is preferably 50% or more, and more preferably 90% or more.

### Example 3. Comparison of pain inhibition effect between Zanthoxylum piperitum leaf fractions

The pain inhibition effect was compared between the respective solvent fractions obtained by solvent fractionation of *Zanthoxylum piperitum* leaf ethanol extracts according to Preparation Example 1. That is, for each fraction obtained by sequentially fractionating a 90% ethanol extract with normal hexane, dichloromethane, ethyl acetate, normal butanol and water, the pain inhibition effect was measured using an acetic acid-induced pain model, and the results are shown in Table 5 below.

**[Table 5]**

| Pain inhibition percentage of *Zanthoxylum piperitum* leaf solvent fraction | |
|---|---|
| Fraction | Pain inhibition percentage (%)(50 mg/kg, po) |
| Normal hexane fraction | 24 |
| Dichloromethane fraction | 48 |
| Ethyl acetate fraction | 82 |
| Normal butanol fraction | 27 |
| Water fraction | 42 |

Table 5 showed that, among various solvent fractions, the ethyl acetate fraction showed the most excellent pain inhibition effect.

### Example 4. Comparison of pain inhibition effect between ethyl acetate sub-fractions

The pain inhibition efficacy was compared between seven sub-fractions obtained through silica gel column chromatography of the ethyl acetate fraction according to Preparation Example 1. That is, the pain inhibition efficacy of the sub-fractions classified as ZPE1, ZPE2, ZPE3, ZPE4, ZPE5, ZPE6 and ZPE7 was measured using an acetic acid-induced pain model, and the results are shown in Table 6 below.

**[Table 6]**

| Pain inhibition percentage of ethyl acetate sub-fraction | |
|---|---|
| EtOAc sub-fraction | Pain inhibition percentage (%)(100 mg/kg, po) |
| ZPE 1 | 18.2 |
| ZPE 2 | 30.1 |
| ZPE 3 | 31.6 |
| ZPE 4 | 59.5 |
| ZPE 5 | 71.3 |
| ZPE 6 | 82.9 |
| ZPE 7 | 48.3 |

Table 6 showed that, among various ethyl acetate sub-fractions, the sixth sub-fraction (ZPE6) showed the most excellent pain inhibition effect.

### Example 5. Comparison of pain inhibition effect between ZPE6 sub-fractions

The pain inhibition effect was compared between three sub-fractions separated by medium-pressure liquid chromatography from the ZPE6 fraction obtained in Preparation Example 1. That is, the pain inhibition effect of the sub-fractions classified as ZPE6A, ZPE6B and ZPE6C was measured using an acetic acid-induced pain model, and the results are shown in Table 7 below.

**[Table 7]**

| Pain inhibition percentage of ZPE6 sub-fraction | |
|---|---|
| ZPE6 sub-fraction | Pain inhibition percentage (%) (100 mg/kg, po) |
| ZPE6A | 64.1 |
| ZPE6B | 79.4 |
| ZPE6C | 66.2 |

Table 7 showed that, among ZPE6 sub-fractions, the second sub-fraction (ZPE6B) showed the most excellent pain inhibition effect.

### Example 6. Comparison of pain inhibition effect of active ingredients isolated from ZPE6B sub-fraction

Two active ingredients, namely quercetin-3-O-alpha-L-rhamnoside (Quercitrin) and camphorol-3-O-alpha-L-rhamnoside (Afzelin), represented by Formulas 1 and 2, respectively, were isolated and compared with regard to the pain inhibition effect. In other words, the pain inhibition effect of each of quercetin-3-O-alpha-L-rhamnoside (Quercitrin) and camphorol-3-O-alpha-L-rhamnoside (Afzelin) was measured using an acetic acid-induced pain model, and the results are shown in Table 8 below.

**[Table 8]**

| Pain inhibition percentage of active ingredient | |
|---|---|
| active ingredient | Pain inhibition percentage (%)(50 mg/kg, po) |
| ZPE6B | 76.4 |
| Quercitrin | 69.2 |
| Afzelin | 93.6 |

Table 8 showed that the quercetin-3-O-alpha-L-rhamnoside (Quercitrin) and camphorol-3-O-alpha-L-rhamnoside (Afzelin) as effective (active) ingredients represented by Formulas 1 and 2, respectively, exhibited an excellent pain inhibition effect in the acetic acid-induced pain model. In particular, afzelin separated from the ZPE6 sub-fraction exhibited a very potent pain inhibition effect.

The results of Table 8 show that, among the three sub-fractions separated by medium-pressure liquid chromatography (MPLC) in Preparation Example 1, ZPE6B exhibited an excellent pain inhibition effect, and the reason therefor is that ZPE6B contains a large amount of a compound having excellent pain inhibition activity, such as Afzelin.

### Example 7. Comparison in GC-MS analysis between ZPE6A and ZPE6C sub-fractions

Among the three sub-fractions separated in Preparation Example 1, ZPE6A and ZPE6C exhibited lower pain inhibition effects than ZPE6B, but better pain inhibition effects when compared to other fractions or control groups. Table 9 below shows the results of GC-MS analysis to analyze the active ingredients contained in the sub-fractions ZPE6A and ZPE6C.

**[Table 9]**

| Peak No. | Compound | ZPE6A | | ZPE6C | |
|---|---|---|---|---|---|
| | | RT | (Area (%) | RT | Area (%) |
| 1 | 2,4-di-*tert*-butylphenol | 14.540 | 26.69 | 14.540 | 12.09 |
| 2 | Eicosane | 15.974 | 3.99 | 14.288 | 3.67 |
| 3 | Methyl myristate | 17.977 | 4.00 | 17.978 | 4.84 |
| 4 | Methyl palmitate | 20.933 | 32.09 | 20.934 | 31.05 |
| 5 | 1,2-benzenedicarboxylic acid | 21.514 | 6.99 | 21.514 | 9.53 |
| 6 | Methyl stearate | 23.638 | 10.78 | 23.649 | 9.86 |
| 7 | 1,2-benzenedicarboxylic acid dipropyl ester | 28.674 | 15.47 | 28.674 | 17.85 |
| 8 | 4'-hydroxyacetophenone | N/D | N/D | 12.514 | 5.62 |
| 9 | Nonadecane | N/D | N/D | 15.974 | 5.49 |

The GC-MS analysis results of Table 9 showed that the ZPE6A or ZPE6C fraction contains 4'-hydroxyacetophenone represented by the following Formula 3, 2,4-di-tert-butylphenol represented by the following Formula 4, and 1,2-benzenedicarboxylic acid represented by the following Formula 5.

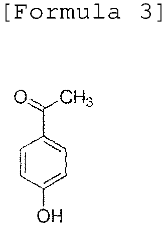

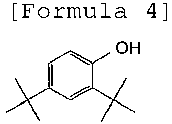

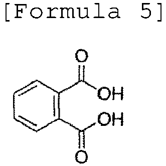

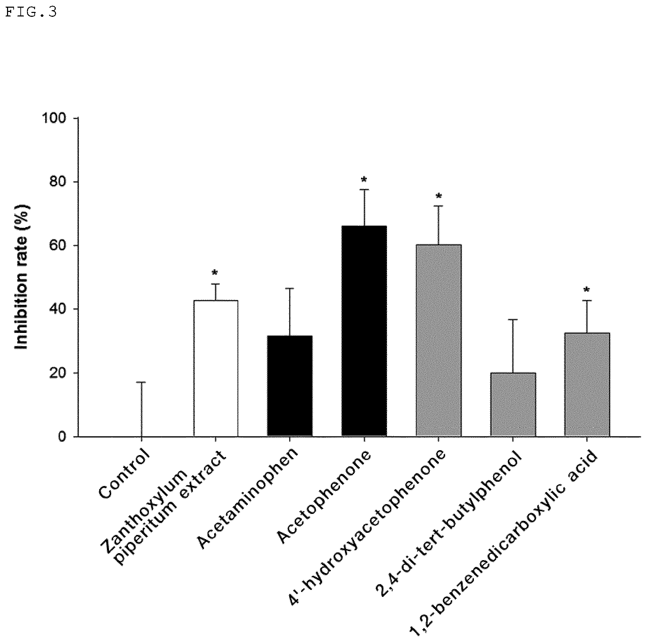

In addition, the pain inhibition effects of 4'-hydroxyacetophenone, 2,4-di-tert-butylphenol and 1,2-benzenedicarboxylic acid isolated from the ZPE6A or ZPE6C fraction were measured using the acetic acid-induced pain model and the results are shown in FIG. 3.

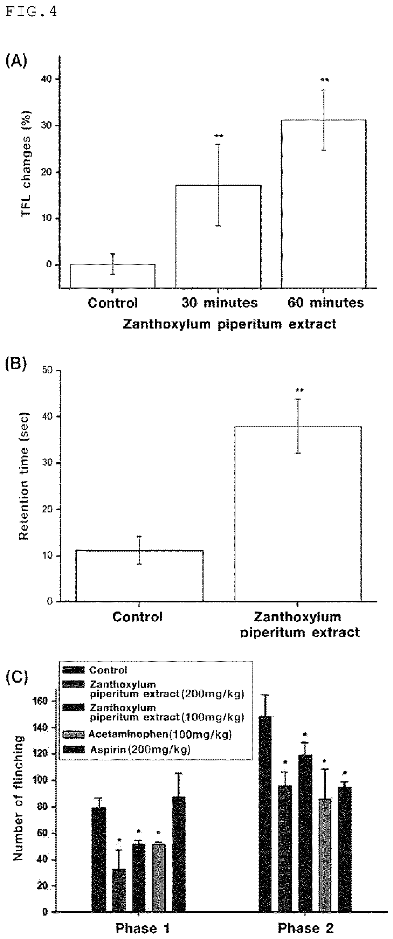

As can be seen from FIG. 3, 4'-hydroxyacetophenone, 2,4-di-tert-butylphenol and 1,2-benzenedicarboxylic acid have excellent pain inhibition effects, and among them, 4'-hydroxyacetophenone had the most excellent pain inhibition effect.

### Example 8. Comparison in content of active compounds depending on extraction conditions

In the process of obtaining the *Zanthoxylum piperitum* leaf extract according to Preparation Example 1, each extract was obtained while changing the extraction solvent and temperature conditions. For each extract, the contents of the active compounds (quercitrin, afzelin) were analyzed by HPLC, and the pain inhibition percentage was measured using an acetic acid-induced pain model, and the results are shown in Table 10 below.

### [Extraction conditions]

Condition 1) 90% ethanol extract (non-heated),
Condition 2) 90% ethanol extract (heated),
Condition 3) a substance of the 90% ethanol extract from which an emulsion was removed by centrifugation using a heating method.
Condition 4) a fraction of the 90% ethanol extract fractionated with dichloromethane
Condition 5) a residue of the fraction of the 90% ethanol extract fractionated with dichloromethane
Condition 6) a normal hexane extract
Condition 7) an ethanol extract obtained by extracting the normal hexane extract with 90% ethanol
Condition 8) a dichloromethane extract
Condition 9) an ethanol extract obtained by extracting the dichloromethane extract with 90% ethanol

**[Table 10]**

| Comparison in pain inhibition percentage of extract and content of active compound depending on extraction condition | | | | | |
|---|---|---|---|---|---|
| Extraction condition | Sample name | Yield (%) | Pain inhibition percentage (%) (50 mg/kg, po) | Active (area %, RT compound 25 min) | |
| | | | | Quercitrin | Afzelin |
| Condition 1 | EtOH extract-non-heating | 5.1 | 45.5 | 36.47 | 12.85 |
| Condition 2 | EtOH extract-heating | 6.8 | 75.2 | 56.44 | 4.68 |
| Condition 3 | Substance of Condition 2 extract from which emulsion is removed | 5.7 | 74.5 | 58.02 | 4.05 |
| Condition 4 | CH₂Cl₂ fraction of ethanol extract | 2.2 | 41.8 | 2.60 | 7.88 |
| Condition 5 | Residue of Condition 4 | 3.6 | 60 | 66.47 | 15.36 |
| Condition 6 | Normal hexane extract | 1.1 | 76.4 | 0.41 | 4.81 |
| Condition 7 | EtOH extract of Condition 6 extract | 5.1 | 0 | 49.5 | 14.33 |
| Condition 8 | CH₂Cl₂ extract | 1.7 | 81.8 | 0.37 | 6.01 |
| Condition 9 | EtOH extract of Condition 8 extract | 4.5 | 23.6 | 59.49 | 15.91 |

As can be seen from Table 10, the content of the active compound may vary depending on the extraction condition. As suggested by the present invention, the alcohol extracts obtained through extraction under Conditions 1, 2 and 3 had a very high content of the active compound and effectively inhibited pain.

### Example 9. Analgesic effect of Zanthoxylum piperitum leaf extract

The analgesic efficacy of the 90% ethanol extract of *Zanthoxylum piperitum* leaves obtained in Preparation Example 1 was measured using various animal models.

FIG 4 shows the result of the analgesic effect of the *Zanthoxylum piperitum* leaf extract measured by (a) a tail-flick test, (b) a hot-plate test and (c) a formalin test. As can be seen from FIG 4, the *Zanthoxylum piperitum* leaf extract according to the present invention has an excellent pain inhibition effect. Pain is caused by a pain-inducing stimulus that is transferred through the sensory nerves to the spine and brain. Pain is divided into somatic pain related to the skin, muscles and bones, visceral pain related to the internal organs, and neuropathic pain detected in the sensory nerve area entering the same vertebral ganglion as the nerve that controls the corresponding organ. The test suggested in FIG. 4 indicates that the *Zanthoxylum piperitum* leaf extract has a pain inhibition effect on all of acute pain, chronic pain, pain of the central nervous system and pain of the peripheral nervous system, and has an inhibitory effect on pain due to physical damage, pressure, and stress applied to the skin, muscles, bones, organs and the like, without being limited to the therapeutic effect on inflammation.

### Example 10. Anti-inflammatory effect of Zanthoxylum piperitum leaf extract

The anti-inflammatory effect of 95% ethanol extracts of the *Zanthoxylum piperitum* leaves obtained in Preparation Example 1 was measured using enzyme immunoassay, reverse transcription-PCR analysis and immunoblotting.

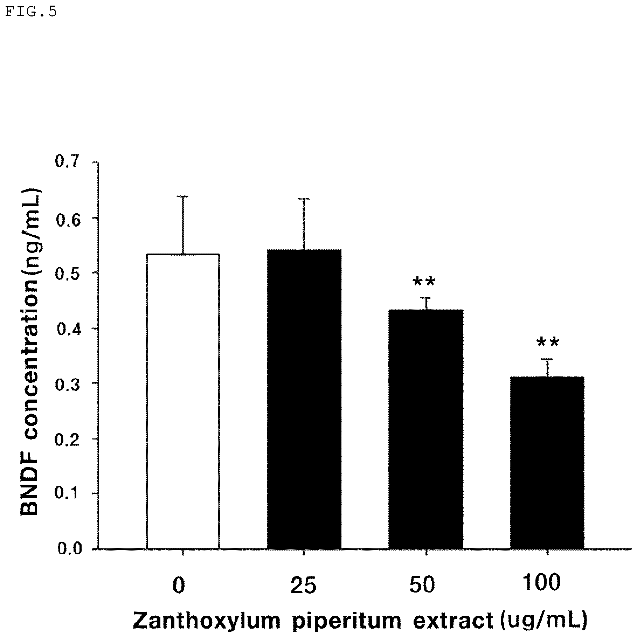

FIG. 5 shows the result of measurement of the expression level of BDNF for amounts of treated *Zanthoxylum piperitum* leaf extract, of 0, 25, 50 and 100 µg/mL using immunoassays. BDNF expression decreased as the amount of *Zanthoxylum piperitum* increased. The result showed that the anti-inflammatory activity was the highest at 100 *µ*g/mL of the *Zanthoxylum piperitum* leaf extract.

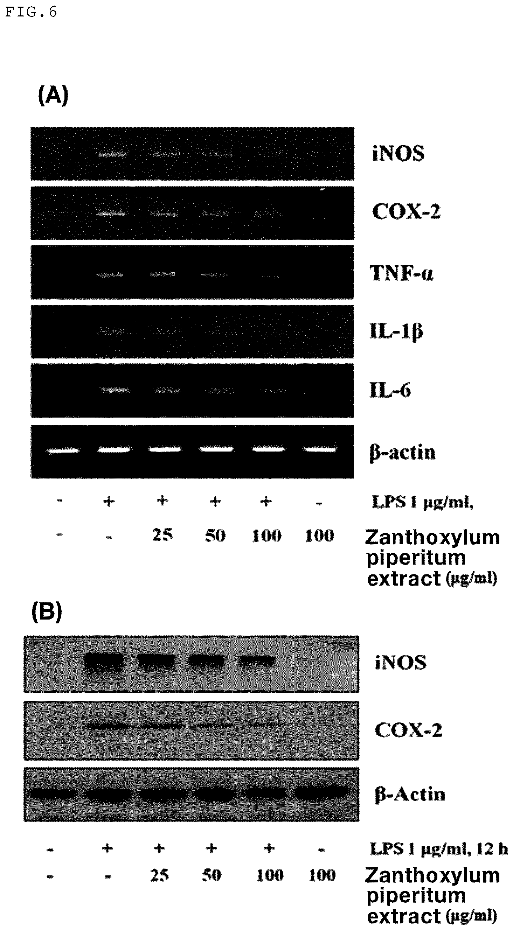

FIG. 6 shows the results of the expression inhibition effects of the inflammatory markers measured using (A) reverse transcription-PCR analysis and (B) immunoblotting. As can be seen from FIG. 6, the group treated with the *Zanthoxylum piperitum* leaf extract effectively inhibited the expression of inflammatory markers of iNOS, COX-2, TNF-α, IL-1β and IL-6 overexpressed by lipopolysaccharide (LPS).

### Example 11. Anti-inflammatory effect of Zanthoxylum piperitum leaf extract in subacute ear edema model

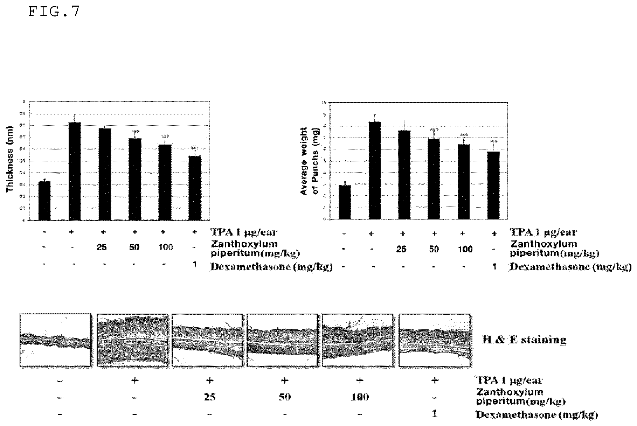

The result of measurement of anti-inflammatory effect after oral administration of the *Zanthoxylum piperitum* leaf extract at concentrations of 25, 50 and 100 mg/kg in a mouse model having ear edema induced by TPA is shown in FIG. 7.

Both the thickness and the weight of the ear were significantly increased by TPA, and the thickness and weight of ear edema were effectively reduced by oral administration of the *Zanthoxylum piperitum* leaf extract. The anti-inflammatory effect increased as the amount of the treated *Zanthoxylum piperitum* leaf extract increased. The result of tissue immunological analysis through immunohistochemistry showed that the *Zanthoxylum piperitum* leaf extract effectively inhibited the penetration of immune cells into tissue.

### Example 12: Effect of Zanthoxylum piperitum leaf extract on treatment of rheumatoid arthritis

As one of the methods for evaluating the therapeutic effect of rheumatoid arthritis, the antirheumatic efficacy of each test substance was measured in a collagen-induced arthritis animal model. The experimental animals herein used were 5-week-old DBA/1J mice purchased from Central Laboratory Animals (Seoul) and acclimated in a breeding room maintained at a humidity of 50% and a temperature of 24 to 26°C for one week. Feed and water were supplied for free intake. In order to induce arthritis, type 2 collagen and CFA (complete Freund's adjuvant) were mixed to obtain an emulsion. 200 µL of the emulsion was injected subcutaneously (id) into the lower part of the tail 2 to 3 cm away from the torso of DBA/1J mice, and the same amount of the emulsion was secondarily injected in 150 µL into the lower part of the tail in the same manner as in the primary injection 21 days after exposure to a primary antigen. A positive control group was orally administered with dexamethasone at a dose of 1 mg/kg from the 22^{nd} day after primary antigen exposure, the group administered with the *Zanthoxylum piperitum* leaf extract was orally treated at 100 mg/kg for 3 weeks, rheumatoid arthritis lesions were visually observed and graded, and the arthritis alleviation efficacy was measured as a clinical score.

The visual clinical score measured by the method is shown in Table 11 below.

**[Table 11]**

| Measurement of visual clinical score in collagen-induced arthritis animal model | | | | | | |
|---|---|---|---|---|---|---|
| Group | No. | Right front leg | Left front leg | Right back leg | Left back leg | Total |
| Control group | 1 | 3 | 3 | 1 | 4 | 11 |
| | 2 | 3 | 3 | 4 | 4 | 14 |
| | 3 | 3 | 3 | 2 | 1 | 9 |
| | 4 | 3 | 3 | 4 | 4 | 14 |
| | 5 | 3 | 3 | 3 | 2 | 11 |
| | 6 | 3 | 1 | 4 | 4 | 12 |
| Positive control group | 1 | 1 | 2 | 1 | 1 | 5 |
| | 2 | 1 | 1 | 1 | 2 | 5 |
| | 3 | 3 | 3 | 1 | 1 | 8 |
| | 4 | 3 | 2 | 1 | 1 | 7 |
| | 5 | 2 | 1 | 1 | 2 | 6 |
| | 6 | 2 | 2 | 1 | 1 | 6 |
| | 7 | 2 | 3 | 1 | 1 | 7 |
| Group administered with *Zanthoxylum piperitum* extract | 1 | 1 | 1 | 1 | 2 | 5 |
| | 2 | 1 | 1 | 1 | 3 | 6 |
| | 3 | 1 | 1 | 3 | 3 | 8 |
| | 4 | 1 | 2 | 1 | 3 | 7 |
| | 5 | 2 | 1 | 4 | 2 | 9 |
| | 6 | 3 | 3 | 2 | 4 | 12 |
| | 7 | 1 | 1 | 4 | 3 | 9 |

As shown in Table 11, the average score of the control group was 11.8, whereas the average score of the group administered with the *Zanthoxylum piperitum* leaf extract was very low, specifically 8. When taking into consideration the fact that the average score of the positive control group, i.e., the group administered with dexamethasone, was 6.3, the *Zanthoxylum piperitum* leaf extract was found to be effective as a therapeutic agent for rheumatoid arthritis.

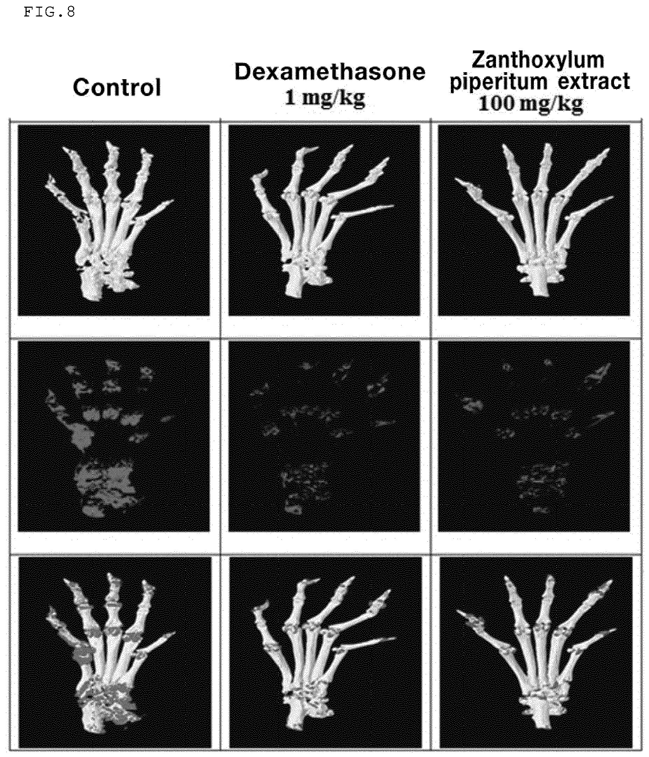

In addition, FIG. 8 shows the results of analysis of the ankle joint of the hind paw on a 3D image using micro-CT. In the control group, the bone shape in the joint is changed irregularly, which means that destruction of the bone due to inflammation progressed considerably. On the other hand, it can be seen from the 3D image that the group administered with the *Zanthoxylum piperitum* leaf extract exhibited a significant reduction in bone destruction and deformation and substantially the same therapeutic effect on arthritis as the group administered with dexamethasone.

### Example 13. Therapeutic effect in osteoarthritis-induced animal model

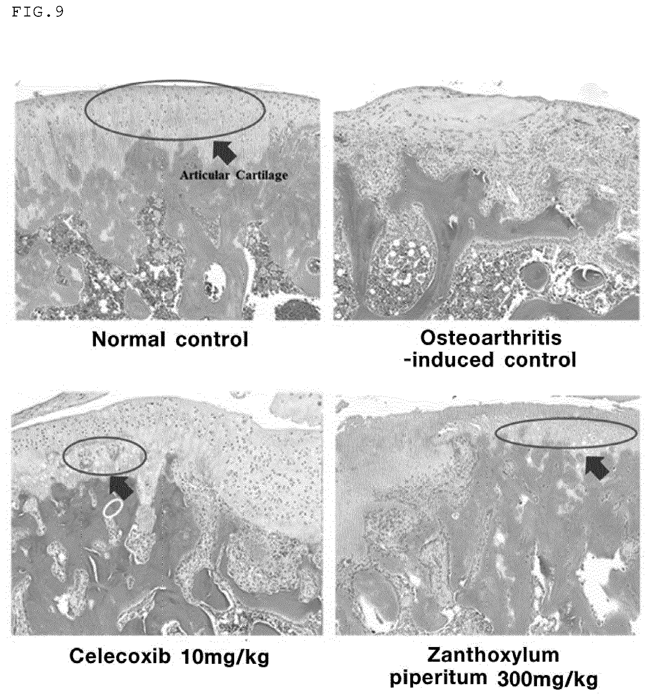

As one of the methods for evaluating the effect of treating osteoarthritis, the alleviation effect of the test substance on collagenase-induced arthritis was measured. The experimental animals herein used were 5-week-old SD rats purchased from Central Laboratory Animals (Seoul) and acclimated in a breeding room maintained at a humidity of 50% and a temperature of 24 to 26°C for one week. Feed and water were supplied for free intake. In order to induce arthritis, 4 mg/mL of type 2 collagenase was injected into the articular capsule of the right joint of each rat in an amount of 0.05 mL (0 day) and the test started 4 days later. After the collagenase injection, a normal control group administered with distilled water, the osteoarthritis induction control group, a positive control group administered with celecoxib (10 mg/kg) and an experimental group administered orally once daily with the *Zanthoxylum piperitum* leaf extract (300 mg/kg) were prepared. After 7 weeks, the joint part was histopathologically observed, and the results are shown in FIG. 9.

As can be seen from FIG. 9, the group administered with the *Zanthoxylum piperitum* leaf extract exhibited a significant reduction in the inflammation site after 7 weeks and exhibited a more potent effect than the group administered with celecoxib.

### Example 14. Therapeutic effects in osteoarthritis-induced animal model

Osteoarthritis is caused by metabolic abnormalities of cartilage cells, and representative enzymes involved in cartilage damage include matrix metalloproteinases (MMPs) and tissue inhibitors of metalloproteinases (TIMPs). The osteoarthritis animal model induced by administration of MIA (monosodium iodoacetate) causes cartilage damage of the knee joint by interfering with the metabolism of cartilage cells and exhibits symptoms similar to those of osteoarthritis.

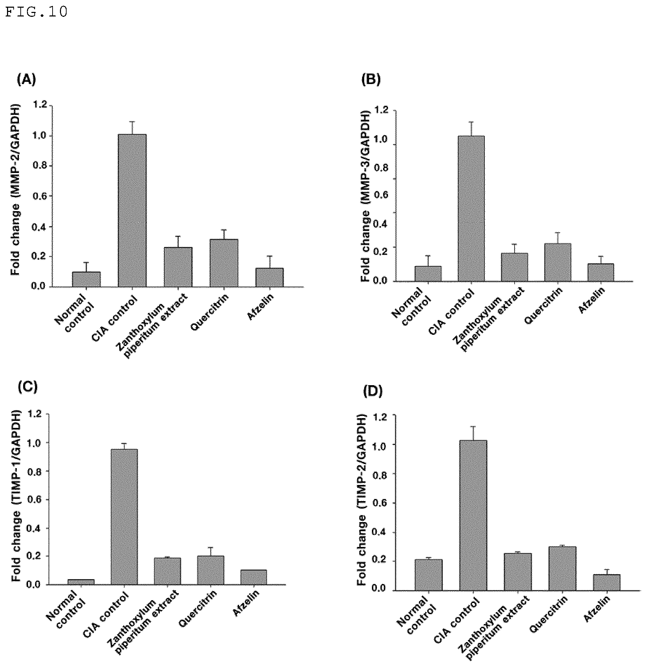

FIG. 10 shows the results of identification of changes in biochemical indicators of MMP-2, MMP-3, TIMP-1 and TIMP-2 in an MIA-induced osteoarthritis animal model. As can be seen from FIG. 10, the *Zanthoxylum piperitum* leaf extract and the active compounds (quercitrin, afzelin) had an excellent effect of treating osteoarthritis, and in particular, afzelin was found to be excellent in treating osteoarthritis.

### Example 15: Determination of pain inhibitory mechanism of Zanthoxylum piperitum leaf extract and active substances

In order to investigate various inflammation such as arthritis and pain inhibition mechanisms, a PCR sequence was observed using BV-2 cells, which are brain microglia. The experimental groups were respectively administered with *Zanthoxylum piperitum* leaf extract and the active compounds quercitrin and afzelin.

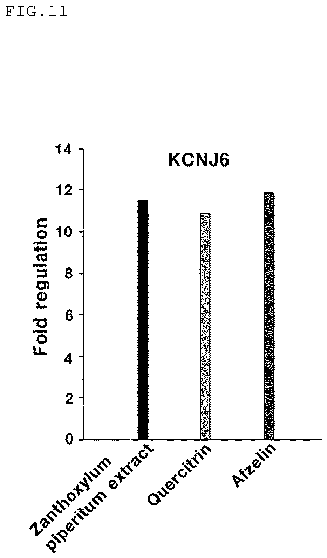

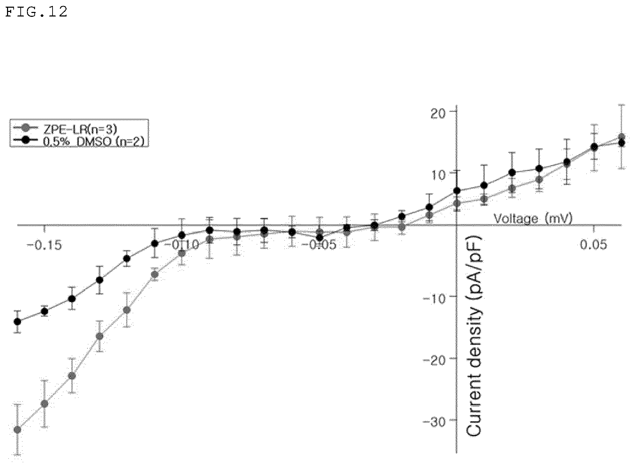

FIG. 11 is a graph showing the expression level of the protein KCNJ6 in BV-2 cells, which are brain microglia after treatment with the *Zanthoxylum piperitum* leaf extract, quercitrin and afzelin. In addition, FIG. 12 shows a graph of potassium (K⁺) current density in BV-2 cells, which are brain microglia.

As can be seen from the results of FIGS. 11 and 12, K⁺ current density increases as the expression level of KCNJ6, a K+ gate regulatory protein, increases. In other words, the pain disease caused by reducing potassium (K⁺) current density can be treated owing to the increased potassium (K+) current density resulting from treatment with the *Zanthoxylum piperitum* leaf extract, quercitrin or afzelin.

### Example 16. Rheumatoid arthritis alleviation effect in collagen-induced animal model (CIA)

The therapeutic effect of rheumatoid arthritis can be identified by treating the collagen-induced animal model (CIA) with the *Zanthoxylum piperitum* leaf extract or active compounds (quercitrin and afzelin), separating NK cells (natural killer cells) and then measuring the expression level of IFN-γ cells.

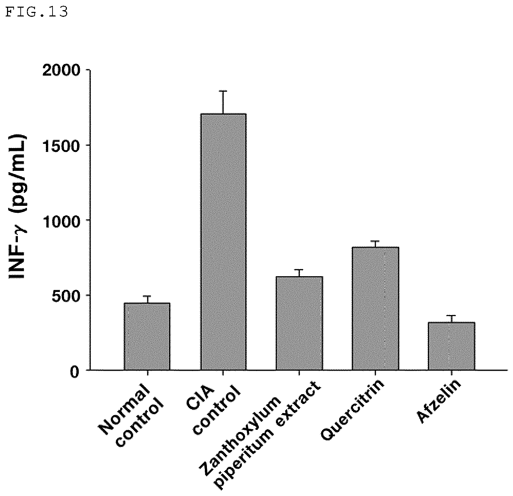

IFN-γ is a cytokine produced in T cells or NK cells by immunostimulation. Collagen-derived animals increased the expression of IFN-γ cells. FIG. 13 is a graph showing IFN-γ production in NK cells measured after treating the collagen-induced animal model (CIA) with the *Zanthoxylum piperitum* leaf extract or active compound (quercitrin, afzelin). As can be seen from FIG. 13, compared to the collagen-induced animal model (CIA), the experimental group treated with the *Zanthoxylum piperitum* leaf extract or the active compound (quercitrin, afzelin) exhibited a significant reduction in the expression level of IFN-γ in NK cells.

### Example 17. Rheumatoid arthritis alleviation effect in collagen-induced animal model (CIA)

The expression level of IL-6 of T-cells and the expression levels of TH17, IL-21 and IL-22 of TH17 cells were measured after treating the collagen-induced animal models (CIA) with the *Zanthoxylum piperitum* leaf extract or active compounds (quercitrin, afzelin) to evaluate the therapeutic effect of rheumatoid arthritis.

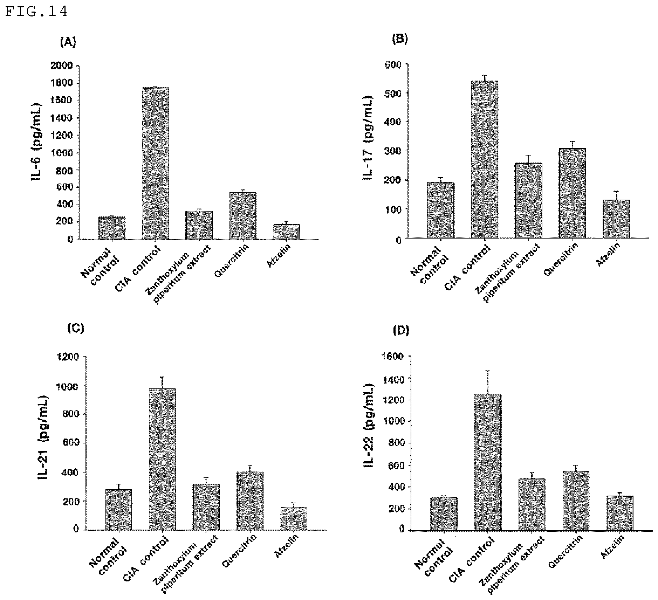

Rheumatoid arthritis is an inflammatory disease accompanied by severe pain in various joints of the human body. Rheumatoid arthritis patients have large amounts of pro-inflammatory cytokines (IL-6, IL-17, IL-21 and IL-22) in their joints. FIG. 14 is a graph showing the expression levels of inflammation-promoting cytokines (IL-6, IL-17, IL-21, and IL-) measured after treating the collagen-induced animal model (CIA) with the *Zanthoxylum piperitum* leaf extract or active compounds (quercitrin, afzelin). As can be seen from FIG. 14, compared to the collagen-induced animal model (CIA), the experimental group treated with the *Zanthoxylum piperitum* leaf extract or the active compound (quercitrin, afzelin) decreased the expression level of IL-6 secreted from T cells, and significantly reduced the expression levels of IL-17, IL-21 and IL-22 secreted from TH17 cells.

### Example 18. Autoimmune therapeutic effects

Inhibition of inflammation-promoting cytokines has been reported to inhibit autoimmune diseases such as atopy. Thus, in the present embodiment, the mast cell RBL-2H3 was treated with the *Zanthoxylum piperitum* leaf extract or active compounds (quercitrin, afzelin) to evaluate the secretion inhibition ability of β-hexosaminidase. That is, the effect of inhibiting mast cell degranulation by the *Zanthoxylum piperitum* leaf extract or the active compound (quercitrin, afzelin) was found. The results are shown in Table 12 below.

**[Table 12]**

| Treatment amount | Effect of inhibiting degranulation (% inhibition) | | | |
|---|---|---|---|---|
| (µg/mL) | Ketotifen | *Zanthoxylum piperitum* extract | Quercitrin | Afzelin |
| 100 | 43.9 ± 1.45 | 30.8 ± 0.65 | 22.2 ± 0.32 | 40.9 ± 0.98 |
| 50 | 32.8 ± 1.04 | 16.2 ± 0.48 | 19.8 ± 0.28 | 41.3 ± 1.67 |
| 25 | 20.2 ± 0.84 | 12.8 ± 0.39 | 14.3 ± 0.11 | 39.4 ± 1.06 |
| 12.5 | 13.8 ± 0.88 | 2.7 ± 1.20 | 6.3 ± 0.13 | 25.1 ± 1.68 |

As can be seen from Table 12, the present invention exhibits a better degranulation inhibitory effect on RBL-2H3 cells than a ketotifen drug known as an antihistamine. At a treatment dose of 100 µg/mL, the *Zanthoxylum piperitum* leaf extract had about 70% degranulation inhibitory activity, quercitrin had about 50% degranulation inhibitory activity, and afzelin had about 93% degranulation inhibitory activity compared to the ketotifen drug. At a treatment dose of 25 µg/mL, the *Zanthoxylum piperitum* leaf extract had about 63% degranulation inhibitory activity, quercitrin had about 70% degranulation inhibitory activity, and afzelin had about 195% degranulation inhibitory activity compared to the ketotifen drug.

### [Reference Example]

The present invention is directed to a pharmaceutical composition or a health food composition based on the pain-inhibitory and anti-inflammatory effects of the *Zanthoxylum piperitum* leaf extract. In addition, the prior art, Korean Patent No. 10-1363311, discloses the therapeutic and prophylactic efficacies on ulcerative colitis of a *Zanthoxylum schinifolium* extract.

*Zanthoxylum piperitum* trees and *Zanthoxylum schinifolium* trees are plants belonging to the *Zanthoxylum sp.* and have been commonly considered to be almost the same plant. In this reference example, a variety of analysis results demonstrated that the leaf extract of *Zanthoxylum piperitum* and the fruit extract of *Zanthoxylum schinifolium* are distinct extracts that contain substantially different ingredients.

### (1) Comparison of GC-MS analysis between leaf extract of Zanthoxylum piperitum trees and fruit extract of Zanthoxylum schinifolium trees

The leaves of *Zanthoxylum piperitum* and the fruits of *Zanthoxylum schinifolium* were extracted with a 90% ethanol aqueous solution, and each extract was analyzed by GC-MS. The following Table 13 shows the results of the GC-MS analysis of the leaf extract of *Zanthoxylum piperitum* and the fruit extract of *Zanthoxylum schinifolium.*

**[Table 13]**

| Item | Ingredient | Relative content (%) | | | |
|---|---|---|---|---|---|
| | | *Zanthoxylum piperitum* leaf extract | | *Zanthoxylum schinifolium* fruit extract | |
| | | RT | Area | RT | Area |
| 1 | 2-(2-methyl-12-propen-1-yl)-3-methylthiirane | 5.994 | 0.2 | | |
| 2 | Benzothiazole | 9.406 | 2.87 | | |
| 3 | 2-undecanone | 10.59 | 0.61 | | |
| 4 | 2-butenal | 11.8 | 1.02 | | |
| 5 | 1-deuteriomethyloctane | 12.96 | 0.74 | | |
| 6 | Pent-2-enoic acid ethyl ester | 13.05 | 1.78 | | |
| 7 | 1*H*-1,2,4-triazole | 13.75 | 0.54 | | |
| 8 | 2-tridecanone | 14.24 | 22.97 | | |
| 9 | Phenol | 14.54 | 1.42 | | |
| 10 | Hexanoic | 15.02 | 1.43 | | |
| 11 | 3-*t*-butyl-1-methyl-2-pizozolin-5-one | | | 15.68 | 0.36 |
| 12 | 2,6-octadien-1-ol, 3,7-dimethylacetate | | | 15.84 | 0.63 |
| 13 | Caryophyllene | 15.92 | 0.4 | | |
| 14 | (*trans*)-2-azidocyclopentan-1-ol | 15.99 | 0.84 | | |
| 15 | Methyl-beta-*d*-galactopyranoside | 16.57 | 1.07 | | |
| 16 | Decanoic acid | 16.65 | 1.53 | | |
| 17 | Tridecane | 17.27 | 1.53 | | |
| 18 | 2-Pentadecanone | 17.58 | 15.5 | | |
| 19 | Hydroxy-alpha-terphenyl acetate 2,6-dimethyl-3,5,7-octatrien-2-ol | | | 18.58 | 0.55 |
| 20 | (-)-Loliolide | 18.78 | 1.3 | | |
| 21 | 7*H*-furo[3,2-g]-benzopyran-7-one | 19.7 | 3.93 | | |
| 22 | Psoralen | 19.74 | 6.43 | | |
| 23 | Caffeine | 19.92 | 3.51 | | |
| 24 | 1-8-methadien-4-ol | | | 20.14 | 0.54 |
| 25 | 2-hexadecen-1-ol | 20.32 | 0.88 | | I |
| 26 | 2-dodecanone | 20.6 | 0.64 | | |
| 27 | Hexadecanoic acid | 20.94 | 1.77 | | |
| 28 | 3-7-dimethyl-3-(4-hydroxyphenyl)octa-1 | 21.06 | 0.74 | | |
| 29 | (4,4-dimethyl-2,4,5,6-tetrahydro-1*H*-inden-2-yl)acetic acid | 21.45 | 0.77 | | |
| 30 | 1,2-benzenedicarboxylic acid | 21.51 | 0.53 | | |
| 31 | Octadecanoic acid | 21.88 | 0.67 | | |
| 32 | Isopropyl phenylacetate | 21.93 | 1.03 | | I |
| 33 | 3,7,11-trimethyl-2,4,10-dodecatrien-1-ol | 22.14 | 2.23 | | |
| 34 | 7*H*-furo[3,2-g]-benzopyran-7-one-4-methoxy | 22.94 | 1.04 | | |
| 35 | Ethyl linoleate | 23.24 | 0.44 | | |
| 36 | Ethyl 9,12,15-octadecatriennoate | 23.33 | 1.38 | | |
| 37 | 2-hexadecen-1-ol | 23.48 | 2.04 | | |
| 38 | 4*H*-cyclopenta[*c*]furan | 24.1 | 2.27 | | |
| 39 | Cyclohexene | 24.19 | 0.76 | | |
| 40 | 4,5-dihydro-3-ethyl-5-methyl-1,2,4-triazine | 24.3 | 0.58 | | |
| 41 | Neoherculin benzene acetic acid | 24.45 | 2.05 | | |
| 42 | Neoherculin cyclopentane carboxylic acid | 24.53 | 1.22 | | |
| 43 | 17,11,15-trimethyl, 3-methylene-1-hexadecene | | | 26.44 | 0.22 |
| 44 | 7*H*-furo[3,2-*g*][1]benzopyran-7-one | 26.56 | 0.78 | | |
| 45 | 3- (4-fluoro-3-methylphenyl)propylamine | | | 27.47 | 0.32 |
| 46 | 1-methyl-2-(indol-3'-yl)-benzimidazole | | | 28.21 | 1.08 |
| 47 | Propane, 1-(benzyloxy)-2,3-epoxy | | | 28.5 | 1.43 |
| 48 | 1,2-benzenedicarboxylic acid | 28.67 | 1.2 | | |
| 49 | 2,6,9,11-dodecatetranin-1-carboxylic acid | | | 28.69 | 0.24 |
| 50 | 1,4-cyclooctadiene, 6-bromo | | | 28.81 | 0.75 |
| 51 | 9-amino-6*H*-benzo[*c*]chromen-8,10-dicarbonitrile | | | 28.88 | 1.34 |
| 52 | Hexadecanoic acid | | | 28.93 | 0.75 |
| 53 | 1- (1'-methoxycyclopropyl)-6,6-dimethyl-2,4-cyclooctadien-1-ol | | | 29.49 | 0.44 |
| 54 | Hexadecanoic acid, ethyl ether | | | 29.58 | 0.34 |
| 55 | 2-cyclohexene-1-carboxaldehyde | | | 29.67 | 0.71 |
| 56 | 4,6-dimethylindane | | | 30.96 | 8.56 |
| 57 | 2,2-deuteriobenz[b]quinuclidine | | | 31.67 | 11.02 |
| 58 | 9,10*H*-9-cyanophenanthrene | | | 32.04 | 3.62 |
| 59 | Neoherculin 5-(propen-2-yl)-1,3,7-nonatriene | | | 32.85 | 6.49 |
| 60 | Piperidinyl-(1-oxopropyl)-(cas) | | | 33.03 | 1.29 |
| 61 | 5,5'-(tetrahydro-1*H*,3*H*-furo[3,4-*c*]furan-1,4-diyl)bis-1,3-benzodioxol | 34.77 | 1.67 | | |
| 62 | 5,5 '-(tetrahydro-1*H*,3*H*-furo[3,4-*c*]furan-1,4-diyl)-bis-1,3-benzodioxol-asarinine | 34.84 | 4.11 | | |
| 63 | (1*R*)-(-)-myrtenal | | | 35.24 | 22.12 |
| 64 | 1-methyl-3-(prop-2'-en-1-yl)pyrrolidin-2-one | | | 35.4 | 6.92 |
| 65 | 2-propanone-1-hydroxy-1-phenyl | | | 35.68 | 17.78 |
| 66 | a-acetyl-benzyl alcohol | | | 35.74 | 3.91 |
| 67 | Benzene, 1-methoxy-4-octyl | | | 36.07 | 0.98 |
| 68 | 2-(3-carbomethoxyphenyl)-1,3-dioxolane | 36.76 | 1.6 | | |
| 69 | Benzene, 1-cyclopropylmethyl-4-(1-methylethyl) | | | 37.45 | 0.4 |
| 70 | Alpha-guaiene | | | 37.56 | 0.44 |
| 71 | Cyclopentane carboxylic acid | | | 37.94 | 0.66 |
| 72 | 3-buten-1-ol, 4-chloro-2-methyl-1-phenyl-acetylphenylcarbinol | | | 38.36 | 0.55 |
| 73 | Bis(*p*-phenoxyphenyl)-7-(3,4-methy lenedioxy) -tetrahydrobenzofuranone | | | 41.25 | 0.41 |
| 74 | Sesamin 5,5'-(tetrahydro-1-*H*,3*H*-furo[3,4-*c*] furan-1,4,-diyl) | | | 47.56 | 0.21 |
| 75 | Indole, 5-methyl-2-phenyl-2-ethylacridine | | | 48.42 | 0.35 |
| 76 | Stigmast-5-en-3-ol | | | 49.78 | 0.71 |

As shown in Table 13, the leaf extract of *Zanthoxylum piperitum* and the fruit extract of *Zanthoxylum schinifolium* have almost no ingredients in common, and there is a clear difference in the composition of the ingredients.

In addition, in order to determine the analgesic effect of the leaf extract and fruit extract of *Zanthoxylum piperitum* and the fruit extract of *Zanthoxylum schinifolium* on the peripheral nervous system and the central nervous system, the analgesic effect was measured using a formalin test, and the results are shown in Table 14 below.

**[Table 14]**

| Comparison in analgesic effect between *Zanthoxylum piperitum* leaf and fruit extracts and *Zanthoxylum schinifolium* fruit extract | | | |
|---|---|---|---|
| Experimental group | Administration concentration (mg/kg) | Inhibition (%) | |
| | | Phase 1 | Phase 2 |
| Control group | CMC 0.5% | - | - |
| Acetaminophen | 100 | 35.2 | 41.7 |
| *Zanthoxylum piperitum* leaves | 100 | 35.2 | 22.6 |
| *Zanthoxylum piperitum* fruits | 100 | 16.6 | 10.8 |
| *Zanthoxylum schinifolium* fruits | 100 | 14.9 | 15.5 |

As can be seen from Table 14 above, the effect of the *Zanthoxylum piperitum* leaf extract is significantly better than the *Zanthoxylum piperitum* fruit extract or the *Zanthoxylum schinifolium* fruit extract.

### [Formulation Example]

In the following formulation example, a preparation example of a pharmaceutical composition or health food composition containing the extract, fraction or active compound (quercitrin and afzelin or a mixture thereof) of the present invention as an effective (active) ingredient will be described. The following formulation example is provided only for better understanding, and should not be construed as limiting the scope of the present invention.

### Formulation Example 1. Preparation of pharmaceutical composition

### (1) Preparation of Tablet

10 g of povidone was dissolved in 50 g of ethanol to prepare a binder solution, and then 150 g of the effective (active) ingredient was added to the binder solution, followed by stirring to realize homogeneity. 130 g of silicified microcrystalline cellulose, 15 g of croscarmellose sodium and 20 g of colloidal silicon dioxide were mixed, and then the binder solution which had been stirred homogeneously with the active ingredient was added thereto, followed by a granulation process, and the resulting granules were dried and sized. 15 g of crospovidone was mixed with the prepared granules, and 3.5 g of colloidal silicon dioxide and 6.5 g of magnesium stearate were added thereto, followed by a lubrication process to obtain a tablet.

### (2) Preparation of Tablet

19 g of povidone was dissolved in 50 g of ethanol to prepare a binder solution, and then 150 g of the active ingredient was added to the binder solution, followed by stirring to realize homogeneity. 143 g of silicified microcrystalline cellulose, 15 g of croscarmellose sodium and 20 g of Neusilin were mixed, and then the binder solution which had been stirred homogeneously with to the active ingredient, was added thereto, followed by a granulation process, and the resulting granules were dried and sized. 15 g of crospovidone was mixed with the prepared granules, and 5 g of colloidal silicon dioxide and 8 g of magnesium stearate were added thereto, followed by a lubrication process to obtain a tablet.

### (3) Preparation of Tablet

12.5 g of povidone was dissolved in 50 g of ethanol to prepare a binder solution, and then 150 g of the active ingredient was added to the binder solution, followed by stirring to realize homogeneity. 62.5 g of silicified microcrystalline cellulose, 65 g of a lactose hydrate, 15 g of croscarmellose sodium and 20 g of colloidal silicon dioxide were mixed, and then the binder solution which had been stirred homogeneously with to the active ingredient was added thereto, followed by a granulation process, and the resulting granules were dried and sized. 15 g of crospovidone was mixed with the prepared granules, and 3.5 g of colloidal silicon dioxide and 6.5 g of magnesium stearate were added thereto, followed by a lubrication process to obtain a tablet.

### (4) Preparation of Tablet

19 g of povidone was dissolved in 33.5 g of ethanol to prepare a binder solution, and then 150 g of the active ingredient was added to the binder solution, followed by stirring to realize homogeneity. 105 g (amount equivalent to the amount of ethanol contained in the main component) of silicified microcrystalline cellulose was added, 17.5 g of croscarmellose sodium, and 20 g of Neusilin were mixed, and then the binder solution which had been stirred homogeneously with to the active ingredient was added thereto, followed by a granulation process, and the resulting granules were dried and sized. 17.5 g of crospovidone and 33 g of silicified microcrystalline cellulose were mixed with the prepared granules, and 7.5 g of colloidal silicon dioxide and 8 g of magnesium stearate were added thereto, followed by a lubrication process to obtain a tablet.

### (5) Preparation of coated tablet

Coated tablets were prepared from 755 mg of the tablets prepared in Formulation Examples 1 to 4 (containing 300 mg of the active ingredient) using a conventional pen-coating system (Opadry™, Opadry200™ or Opaglos™).

### Formulation Example 2. Preparation of health food

### (1) Preparation of beverage

1,000 mg of the active ingredient, 1,000 mg of citric acid, 100 g of oligosaccharide and 1 g of taurine were mixed, and purified water was added to the resulting mixture to adjust the total volume to 900 ml. After stirring and heating at 85°C for about 1 hour, the resulting solution was filtered and injected into a sterile 2L container, followed by sealing and sterilizing, to prepare a beverage.

### [Experimental Example]

### Experimental Example 1. Measurement of disintegration of tablets

755 mg (containing 300 mg of active ingredient) of 12 tablets prepared in (5) of Preparation Example 1 were prepared. The number of disintegrated tablets was measured at intervals of 5 minutes according to the pharmacopoeia disintegration test method, and the results are shown in Table 15 below. The test was continued until the 12 tablets were completely disintegrated.

**[Table 15]**

| Experimental group | Number of disintegrated tablets | | |
|---|---|---|---|
| | 5 min | 10 min | 15 min |
| Tablet of Formulation Example 5 | 0 | 3 | 9 |

### Experimental Example 2. Measurement of friability of compression

755 mg of the tablet (containing 300 mg of active ingredient) prepared in (5) of Preparation Example 1 was compressed under different hardness conditions, that is, 10-12 kp, 12-14 kp, and 14 kp or more. As a result of measuring the friability of compression, the friability was 0.1% or less at a hardness of 10 kp or more.

### Experimental Example 3. Measurement of physical properties of tablets

In order to determine the fluidity of 755 mg of the tablet (containing 300 mg of the active ingredient) prepared in (5) of Preparation Example 1, bulk density, tapped density, Hausner ratio, compression index and angle of repose were measured. The results are shown in Table 16 below.

**[Table 16]**

| Item | Tablet of Formulation Example 5 |
|---|---|
| Bulk density | 0.501 |
| Tapped density | 0.547 |
| Hausner ratio | 1.093 |
| Compression index | 8.500 |
| Angle of repose (°) | 38.0 |

## Claims

1. A pharmaceutical composition for treating and preventing pain comprising an ethyl acetate fraction obtained by solvent fractionation of an alcohol extract of *Zanthoxylum piperitum* leaves.

2. The pharmaceutical composition according to claim 1, wherein the ethyl acetate fraction comprises, as an active ingredient, at least one compound selected from the group consisting of 4'-hydroxy-acetophenone, 2,4-di-tert-butylphenol, 1,2-benzenedicarboxylic acid, quercetin-3-O-alpha-L-rhamnoside, and camperol-3-O-alpha-L-rhamnoside.

3. A pharmaceutical composition for treating and preventing pain comprising an active fraction obtained by performing the following steps:
a) extracting leaves of *Zanthoxylum piperitum* with at least one solvent selected from C₁₋₄ alcohol and a C₁₋₄ alcohol aqueous solution to obtain an alcohol extract;
b) suspending the alcohol extract with water and then fractionating a resultant suspension with normal hexane, dichloromethane, ethyl acetate and normal butanol in sequence to obtain an ethyl acetate fraction; and
c) subjecting the ethyl acetate fraction to silica gel column chromatography to obtain an active fraction.

4. The pharmaceutical composition according to claim 3, wherein the active fraction comprises, as an active ingredient, at least one compound selected from the group consisting of 4'-hydroxy-acetophenone, 2,4-di-tert-butylphenol, 1,2-benzenedicarboxylic acid, quercetin-3-O-alpha-L-rhamnoside, and camperol-3-O-alpha-L-rhamnoside.

5. The pharmaceutical composition according to claim 3, wherein the solvent used for extraction is a 90 to 95% ethanol aqueous solution.

6. The pharmaceutical composition according to claim 3, wherein the silica gel column chromatography is carried out by elution while changing a volume ratio of ethyl acetate/methanol from 90/10 to 60/40.

7. The pharmaceutical composition according to claim 1 or 3, wherein the pharmaceutical composition is formulated as a tablet.

8. A health food composition for alleviating pain comprising an ethyl acetate fraction obtained by solvent fractionation of an alcohol extract of *Zanthoxylum piperitum* leaves.

9. The health food composition according to claim 8, wherein the ethyl acetate fraction comprises, as an active ingredient, at least one compound selected from the group consisting of 4'-hydroxy-acetophenone, 2,4-di-*tert*-butylphenol, 1,2-benzenedicarboxylic acid, quercetin-3-O-alpha-L-rhamnoside, and camperol-3-O-alpha-L-rhamnoside.

10. A health food composition for alleviating pain comprising an active fraction obtained by performing a fractionation process comprising the following steps:
a) extracting leaves of *Zanthoxylum piperitum* with at least one solvent selected from C₁₋₄ alcohol and a C₁₋₄ alcohol aqueous solution to obtain an alcohol extract;
b) suspending the alcohol extract with water and then fractionating a resultant suspension with normal hexane, dichloromethane, ethyl acetate and normal butanol in sequence to obtain an ethyl acetate fraction; and
c) subjecting the ethyl acetate fraction to silica gel column chromatography to obtain an active fraction.

11. The health food composition according to claim 10, wherein the active fraction comprises, as an active ingredient, at least one compound selected from the group consisting of 4'-hydroxy-acetophenone, 2,4-di-tert-butylphenol, 1,2-benzenedicarboxylic acid, quercetin-3-O-alpha-L-rhamnoside, and camperol-3-O-alpha-L-rhamnoside.

12. The health food composition according to claim 10, wherein the solvent used for extraction is a 90 to 95% ethanol aqueous solution.

13. The health food composition according to claim 10, wherein the silica gel column chromatography is carried out by elution while changing a volume ratio of ethyl acetate/methanol from 90/10 to 60/40.

14. The health food composition according to claim 8 or 10, wherein the pharmaceutical composition is formulated as a tablet.
